# EUROPEAN PATENT APPLICATION

(11) **EP 3 789 914 A1**
(43) Date of publication of application: **10.03.2021**
(21) Application number: 20194895.7
(22) Date of filing: 07.09.2020
(51) Int. Cl.: G06K 9/00

(54) **METHODS AND SYSTEMS FOR AUTOMATED ASSESSMENT OF RESPIRATORY CYTOLOGY SPECIMENS**

(30) Priority: 06.09.2019 IN 201921036047
(71) Applicant: Airamatrix Private Limited, 400 604 Thane (West), Maharashtra (IN)
(72) Inventor: Nitin, Singhal, 400610 Thane (W), Maharashtra (IN); Harshal Chandrakant, Nishar, 400092 Mumbai, Maharashtra (IN); Uttara Yogesh, Joshi, 411048 Pune (IN); Dev Kumar, Das, 400606 Thane (West) (IN); Avaneesh, Meena, 325220 Rajasthan (IN)
(74) Representative: Prüfer & Partner mbB Patentanwälte · Rechtsanwälte

(57) **Abstract**

Methods and systems for automated assessment of respiratory cytology specimens. Embodiments disclosed herein relate to oncology, and more particularly to automated assessment of respiratory cytology specimens for adequacy, diagnosis and treatment decisions for lung carcinoma. A method disclosed herein includes predicting an adequacy of the respiratory cytology specimens using Artificial Intelligence (AI)/deep learning models. The method further includes diagnosing and staging the respiratory cytology specimens on predicting that the respiratory cytology specimens are adequate for further processing. The method further includes generating at least one of an adequate report, an inadequate report, and a diagnosis and staging report.

## Description

### CROSS REFERENCE TO RELATED APPLICATION

This application is based on and derives the benefit of Indian Provisional Application 201921036047 filed on 6th September, 2019, the contents of which are incorporated herein by reference.

### TECHNICAL FIELD

Embodiments disclosed herein relate to oncology, and more particularly to automated assessment of respiratory cytology specimens for diagnosis and treatment of lung carcinoma.

### BACKGROUND

In general, respiratory cytology specimens play an important role in providing diagnostic, prognostic, and therapeutic information for lung/cancer. Aspiration procedures such as, but not limited to, an endobronchial ultrasound-guided transbronchial needle aspiration (EBUS-TBNA) procedure or the like, may be used for assessment of lung lesions by obtaining the respiratory cytology specimens of a patient.

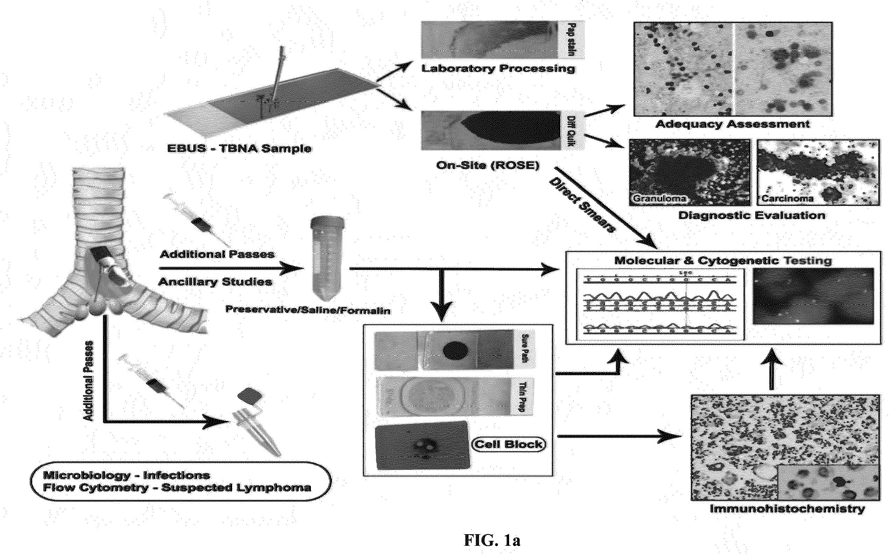

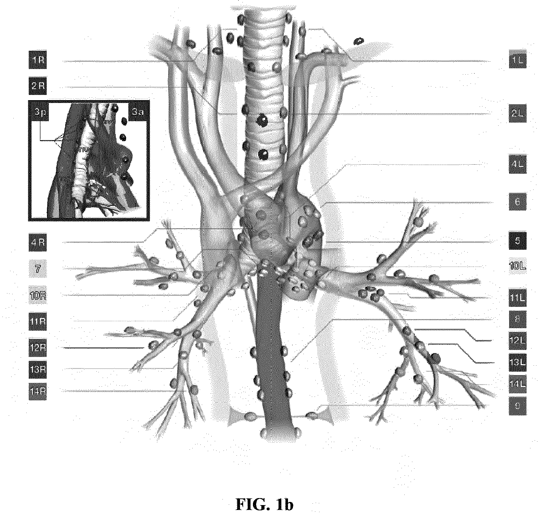

FIGs. 1a and 1b depict a conventional example scenario of assessment of the lung lesions by obtaining the respiratory cytology specimens of the patient using the EBUS-TBNA procedure. The EBUS-TBNA procedure may involve obtaining aspirated specimens from enlarged or FDG avid mediastinal or hilar lymph nodes, or a mass lesion within the mediastinum or lungs (the respiratory cytology specimens). Thereafter, a rapid evaluation process on the obtained EBUS-TBNA specimens needs to be performed for determining adequacy of the EBUS-TBNA specimens. However, the rapid evaluation process is dependent on the on-site availability of an expert pathologist/cytotechnician. In the routine course, the pathologist confirms the adequacy of the EBUS-TBNA specimens using manual analysis under a microscope. In the routine course, once the pathologist has processed and reported the obtained EBUS-TBNA specimens/samples of the patient, results on the adequacy of the EBUS-TBNA specimens may be available only after few days (for example; 2-3 days). In case, the pathologist reports that the samples of the patient are inadequate for evaluation, the aspiration procedure may be repeated. Thus, repetition of the aspiration procedure may result in increased turn-around time for diagnosis and treatment of the lung carcinoma, increased risk of morbidity to the patient, increased hospital visits and expenses, and so on.

The pathologist may confirm the adequacy of the EBUS-TBNA specimens by manually studying smears prepared under the microscope, with a possibility of missing out areas for inspection, which may result in inaccurate assessment of the EBUS-TBNA specimens.

After confirming the adequacy of the EBUS-TBNA specimens, the pathologist may perform diagnosis of malignancy by further detailed manual analysis under the microscope. Such type of diagnosis however requires an expert pathologist.

Thus, in conventional approaches, the assessment of the respiratory cytology specimens involves the manual method of evaluation, which is dependent on the availability and expertise of the pathologist is time consuming and may be highly subjective.

### OBJECTS

The principal object of embodiments herein is to disclose methods and systems for performing automated assessment of respiratory cytology specimens.

Another object of embodiments herein is to disclose methods and systems for predicting adequacy of the respiratory cytology specimens using Artificial Intelligence (AI)/deep learning models.

Another object of embodiments herein is to disclose methods and systems for performing at least one diagnosis using the respiratory cytology specimens on determining that the respiratory cytology specimens are adequate for further diagnosing.

These and other aspects of the embodiments herein will be better appreciated and understood when considered in conjunction with the following description and the accompanying drawings. It should be understood, however, that the following descriptions, while indicating at least one embodiment and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF FIGURES

Embodiments herein are illustrated in the accompanying drawings, throughout which like reference letters indicate corresponding parts in the various figures. The embodiments herein will be better understood from the following description with reference to the drawings, in which:
FIGs. 1a and 1b depict a conventional example scenario of assessment of lung lesions by obtaining respiratory cytology specimens of a patient using an endobronchial ultrasound-guided transbronchial needle aspiration (EBUS-TBNA) procedure;
FIG. 2 depicts an electronic device, according to embodiments as disclosed herein;
FIG. 3a is an example flow diagram depicting a method for predicting adequacy of the respiratory cytology specimens, according to embodiments as disclosed herein;
FIG. 3b is an example flow diagram depicting a method for diagnosing the respiratory cytology specimens, according to embodiments as disclosed herein;
FIG. 4a is an example diagram depicting the respiratory cytology specimen of a patient received as an input for assessment, according to embodiments as disclosed herein;
FIG. 4b is an example diagram depicting a visualization of an output derived by analyzing the input respiratory cytology specimen, according to embodiments as disclosed herein;
FIG. 4c is an example diagram depicting an enhanced visualization of the output derived by analyzing the input respiratory cytology specimen, according to embodiments as disclosed herein;
FIG. 5a is an example diagram depicting an example adequate report, according to embodiments as disclosed herein; and
FIG. 5b is an example diagram depicting an example inadequate report, according to embodiments as disclosed herein.

### DETAILED DESCRIPTION

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

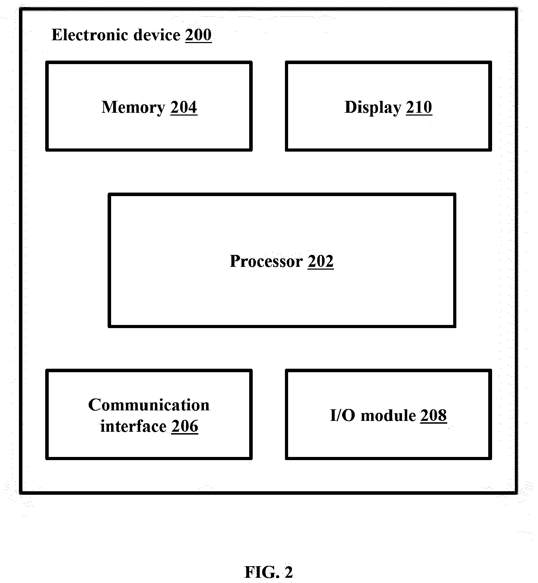

Embodiments herein disclose methods and systems for performing automated assessment of respiratory cytology specimens. Embodiments herein disclose methods and systems for using artificial intelligence (AI)/deep learning models for determining adequacy of the respiratory cytology specimens, and diagnosing and staging the respiratory cytology specimens. Referring now to the drawings, and more particularly to FIGS. 2 through 5b, where similar reference characters denote corresponding features consistently throughout the figures, there are shown embodiments.

FIG. 2 depicts an electronic device 200, according to embodiments as disclosed herein. The electronic device 200 referred herein can be configured to perform automated assessment of respiratory cytology specimens/samples for diagnosing and treatment of lung carcinoma/cancer. The respiratory cytology specimens can be obtained from the respiratory tract in an embodiment herein, the cytologic specimens are obtained from the upper respiratory tract. In an embodiment herein, the cytology specimens are obtained from the lower respiratory tract. Examples of the respiratory cytology specimens can be, but not limited to, endobronchial ultrasound-guided transbronchial needle aspiration (EBUS-TBNA) specimens, Transthoracic Percutaneous Needle Aspiration, Bronchoalveolar lavage, Pleural effusion fluid, Pulmonary arterial catheter-derived blood, Tracheal aspirate, or the like. In an embodiment herein, the electronic device 200 can be, but not limited to, a mobile phone, a smart phone, a tablet, a handheld device, a phablet, a laptop, a computer, a wearable computing device, a medical equipment, an Internet of Thing (IoT) device and so on. In an embodiment herein, the electronic device 200 can be a special-purpose computing system such as, but not limited to, a server, a cloud, a multiprocessor system, a microprocessor based programmable consumer electronic device, a network computer, a minicomputer, a mainframe computer, and so on. In an embodiment herein, the electronic device 200 can be a server coupled with one or more databases (not shown). The server may be a standalone server, or a server on a cloud. In an embodiment herein, the electronic device 200 can be a cloud computing platform, that can be connected to user devices (devices used by a physician/pathologist, a patient, and so on) located in different geographical locations to provide information about the respiratory cytology specimens.

The electronic device 200 includes a processor 202, a memory 204, a communication interface 206, an Input/Output (I/O) module 208, and a display 210. In an embodiment, the electronic device 200 includes imaging sensors, cameras, a scanner, and so on (not shown). In an embodiment, the electronic device 200 may be connected to at least one of the imaging sensors, the cameras, the scanner, and so on externally using a communication network (not shown). Examples of the communication network can be, but is not limited to, the Internet, a wired network (a Local Area Network (LAN), Ethernet and so on), a wireless network (a Wi-Fi network, a cellular network, a Wi-Fi Hotspot, Bluetooth, Zigbee and so on) and so on. The electronic device 200 may also be connected to at least one external entity such as, but not limited to, a server, external databases, and so on using the communication network for accessing information required for performing assessment of the respiratory cytology specimens.

The processor 202 can be at least one of a single processer, a plurality of processors, multiple homogeneous or heterogeneous cores, multiple Central Processing Units (CPUs), microcontrollers, special media, and other accelerators. Further, the plurality of processing units 202 may be located on a single chip or over multiple chips.

In an embodiment, the processor 202 can be configured to perform an automated assessment of the respiratory cytology specimens. The assessment of the respiratory cytology specimens includes predicting adequacy of the respiratory cytology specimens, and diagnosing and staging the respiratory specimens.

In an embodiment, an EBUS-TBNA procedure may be used to obtain the respiratory cytology specimens of the patient. The EBUS-TBNA is essentially a fine needle aspiration performed through the bronchial wall using a bronchoscope and real-time ultrasound guidance. The obtained respiratory cytology specimens are expressed over labeled glass slides for direct smears as an input. The smears can be stained by rapid stains. Examples of the stains can be, but not limited to, Romanowsky stains on unfixed air-dried smears (for example: Diff-Quik, Toluidine Blue, Giemsa stains, or the like), rapid Papanicolaou stains, hematoxylin-eosin stains on wet alcohol-fixed smears, and so on.

For the assessment of the respiratory cytology specimens of the patient, the processor 202 obtains a digital whole slide image of the stained smear(s). In an embodiment, the processor 202 obtains the digital whole slide image of the stained smear using at least one of the image sensors, cameras, and so on present in the electronic device 200. In an embodiment, the processor 202 obtains the digital whole slide image of the stained smear using at least one externally connected entity, such as at least one of the image sensors, cameras, digital whole side image scanners, and so on.

The processor 202 can be configured to analyze the obtained digital whole slide image of the stained smear for predicting the adequacy of the respiratory cytology specimen. In an embodiment, the processor 202 may use at least one method/technique/model such as, Artificial Intelligence (AI) models, deep learning models, and so on for analyzing the digital whole slide image of the stained smear. In an example herein, the deep learning models can be, but not limited to, faster Region-Convolutional Neural Network (R-CNN), mask RCNN, U-Net, SegNet (Semantic Segmentation), VGG-19, ResNet-50, and so on. Embodiments herein are further explained considering the deep learning models as an example for predicting the adequacy of the respiratory cytology specimens, but it may be obvious to a person of ordinary skill in the art that any other suitable neural network models/machine learning models can be considered. In an embodiment, the processor 202 initially trains the deep learning model using annotated data from the pathologist.

The training process involves dividing the annotated data into a training set, a validation set and a test set. In an example, the training set can comprise 80% of the annotated data, the validation set can be 10% of the annotated data, and the test set can be 10% of the annotated data. The processor 202 can apply common data augmentations on the training set. The processor 202 can select the learning model giving best performance on the validation set.

The processor 202 can perform data curation, wherein this involves selecting data from different classes; for example, adequate and inadequate classes in equal proportion. For example, the processor 202 can generate tiles of size 1024 x 1024 at 40x from whole slide cytology specimen. The processor 202 can perform data preprocessing, which involves data augmentation for stain variability. The processor 202 can select a learning model based on training data and complexity. The processor 202 can perform training and perform validation on the non-training data.

The deep learning model may be trained for detection, segmentation, and classification of individual cells/group of cells. The processor 202 may further verify the model by performing a test analysis on the trained deep learning model by passing a single specimen through the trained deep learning model. Test analysis is the validation step which is performed after a fixed number of training iterations to analyze the overfitting or underfitting problem. The trained deep learning model can detect region of interests (ROIs) for a provided sample, perform a boundary/contour segmentation of each detected ROI, and classify each segmented boundary into at least one cell type.

For predicting the adequacy, the processor 202 identifies the individual cells/group of cells based on the obtained digital whole slide image of the stained smear using a suitable image and shape recognition methodology. The processor 202 identifies one or more bounding boxes or ROIs around the identified cells/group of cells. In an embodiment, the processor 202 may use at least one of the faster RCNN and the mask RCNN (the trained deep learning models) for detecting the ROIs based on the obtained stained smear. The processor 202 may further use at least one of the U-Net and the SegNet (the trained deep learning models) for segmenting each ROI and identifying the individual cells/group of cells present in each ROI.

The processor 202 further classifies the identified individual cells/the group of cells into classes/groups/parameters/types such as, but not limited to, lymphocytes, pigmentation/pigment laden macrophages, large cells (a typical/malignant cell), and so on. In an embodiment, the processor 202 can use at least one of the VGG-19 and the ResNet-500 (the trained deep learning models) for classifying the identified individual cells/group of cells into the classes/group. The processor 202 can perform classification using at least one of the VGG-19 or ResNet-500 architectures. The processor 202 extracts features using deep learning architectures with a plurality of convolution layers, followed by inference using fully connected layers. In an embodiment herein, VGG-19 is a 19 layer architecture with 16 convolution layers and 3 fully connected layers.

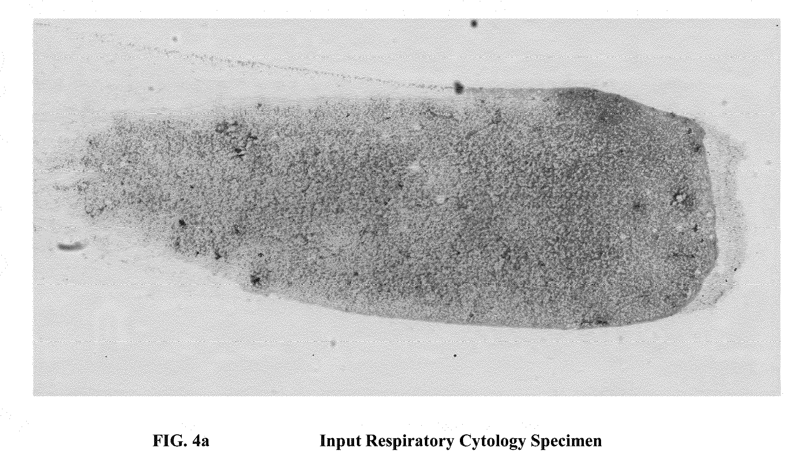

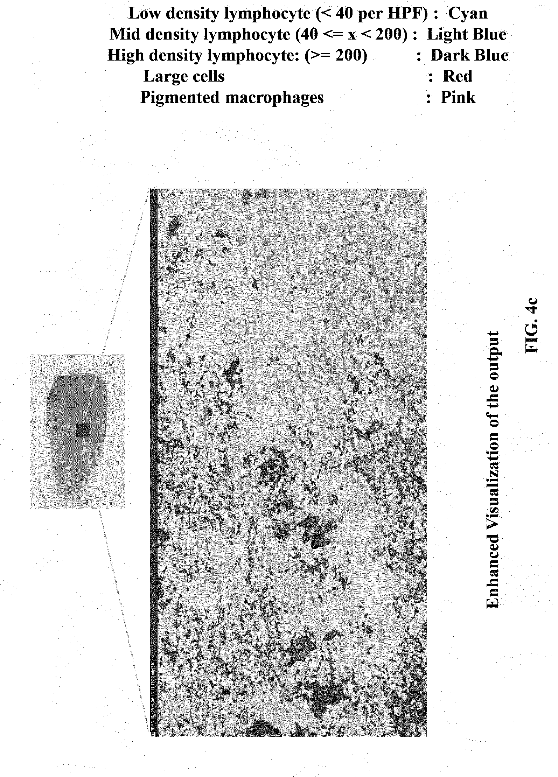

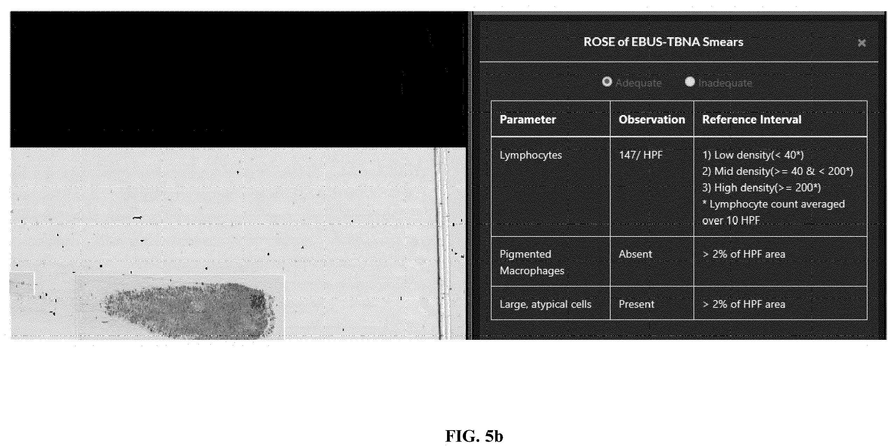

Based on the classification, the processor 202 clusters the individual cells/the group of cells into high power fields (HPFs). HPF can be derived from the field of view of a microscope eye piece. For example, if the scanner resolution is 0.227 µm per pixel at 40x, this corresponds to 536 x 536 Pixels at 10x resolution. The processor 202 then counts the individual cells or the group of cells belonging to each of the HPFs. In an embodiment herein, each of the segmented cell (specifically lymphocyte) is individually counted using connected component labelling. The processor 202 also assigns a grade to each HPF based on the classes/parameters associated with the individual cells/group of cells. FIG. 5b depicts the process of deciding the grades based on preset criteria. The grade can be, but is not limited to low density, medium density and high density. The processor 202 also displays a visualization and localization of the HPFs that are assigned with the low density, the medium density, and the high density grades by assigning a color to each grade in accordance with a color coding scheme (as depicted in the examples in FIGs. 4a, 4a and 4c).

The processor 202 further uses the grades assigned to each HPF to assess/predict the adequacy of the respiratory cytology specimens. The processor 202 predicts the adequacy of the respiratory cytology specimens by identifying, counting, and classifying the relevant entities (such as, lymphocytes) in the graded HPFs. Based on the predicted adequacy, the processor 202 determines if the obtained respiratory cytology specimens are adequate for further diagnosing and staging. In an embodiment herein, the sample is considered as adequate, if more than a pre-defined number of lymphocytes are detected per HPF. For example, the sample is considered as adequate, if there are more than 40 lymphocytes per HPF. In an embodiment herein, the sample is considered as adequate if at least one instance of pigmented macrophages has been detected in the sample. In an embodiment herein, the sample is considered as adequate if at least one instance of large atypical cells has been detected in the sample. On determining that the respiratory cytology specimens are inadequate, the processor 202 generates an inadequate/inadequacy report indicating that the obtained respiratory cytology specimens are inadequate for further diagnosing and staging. On determining that the respiratory cytology specimens are adequate for diagnosing and staging, the processor 202 generates an adequacy/adequate report. The adequate report includes information about at least one of the individual cells/group of cells belonging to the HPFs of the medium density and high density, the classes/parameters/types associated with the individual cells/group of cells, a reference interval, and so on.

The reference interval can depend on the type of cell (lymphocyte, macrophage, large atypical cells, and so on). For example, for a low-density lymphocyte, the reference interval is less than 40 per HPF. For example, for a mid-density lymphocyte, the reference interval is between 40 and 200 per HPF. For example, for a high-density lymphocyte, the reference interval is greater than or equal to 200 per HPF. For example, for pigmented macrophages, the reference interval is greater than 2% of the HPF area. For example, for large atypical cells, the reference interval is greater than 2% of the HPF area.

Based on the generation of the adequate report, the processor 202 can be configured to diagnose and stage the predicted adequate respiratory cytology specimens. In an embodiment, the processor 202 may use at least one method/technique such as, Artificial Intelligence (AI), deep learning, and so on, to diagnose and stage the predicted adequate respiratory cytology specimens. Examples of the deep learning models, can be, but not limited to, VGG-19, ResNet-50, and so on. Embodiments herein are further explained considering the deep learning models for diagnosing the respiratory cytology specimens, but it may be obvious to a person of ordinary skill in the art that any other suitable neural network models/machine learning models can be considered. The processor 202 initially trains the deep learning model for classifying the different cell types received from the adequacy report into malignant (cancer) or benign (noncancerous) classes. The trained deep learning model can act as a binary classification model. The processor 202 may perform a test analysis by passing the adequate report to the binary classification model, which classifies each cell type included in the adequate report into the malignant and benign classes.

The processor 202 classifies each cell type/class associated with the individual cells/group of cells (such as pigment laden macrophages, large (atypical /malignant) cells or the like) that are included in the adequate report into malignant and benign classes using at least one of the VGG-19 and the ResNet-50 (the trained deep learning model). The processor 202 also displays an enhanced visualization and localization of the malignant and benign classes. The processor 202 analyzes the characteristics of the malignant and benign classes to stage the malignant and benign classes. Examples of the characteristics can be, but not limited to, cell boundary, size, shape, morphology, cytoplasm to nucleus ratio, and so on. Staging helps in determining the severity and extent of the disease. Embodiments herein use the morphological and staging related parameter to train a deep learning model for determining dosage, wherein the model can be a simple linear regression model. Staging is performed by extracting one or more morphological features from individual cells/group of cells. These features include, but are not limited to, shape, size, texture, color, and so on.

After diagnosing and staging the predicted adequate respiratory cytology specimens, the processor 202 generates a diagnosis and staging report. The diagnosis and staging report include information about the specimens of malignant and benign classes.

In an embodiment, the processor 202 can be further configured to decide a therapy planning/dosage based on the diagnosis and staging report for further treatment. Based on the stage of the malignant cell, the processor 202 determines an amount of dosage. For example, radiation dosage in chemotherapy can be determined for the further treatment. In an embodiment, the processor 202 may use a deep learning model, which can predict the dosage by creating a linear regression model or a non-linear regression model based on the diagnosing and staging report. For example, consider that there are twenty stages of cancer. The regression model has details of medicine(s) and dosage(s) associated with each stage, which can be used for predicting the dosage. Thus, providing a solution for better patient care by avoiding a repetition of invasive procedures and a manual method of evaluation. The indications of EBUS TBNA are cancer staging and cancer restaging after neo-adjuvant chemotherapy, in addition to diagnosis of mediastinal and central lung masses. Can we frame this accordingly?

The memory 204 stores at least one of the obtained digital whole slide images of the stained smear samples, the adequate report, the inadequate report, the diagnosis and staging report, the therapy planning, and so on. The memory 204 may also store program code/instructions that can be executed on the processor 202 to perform the automated assessment of the respiratory cytology specimens. Further, the memory 204 may include one or more computer-readable storage media. The memory 204 may include non-volatile storage elements. Examples of such non-volatile storage elements may include magnetic hard discs, optical discs, floppy discs, flash memories, or forms of electrically programmable memories (EPROM) or electrically erasable and programmable (EEPROM) memories. In addition, the memory 204 may, in some examples, be considered a non-transitory storage medium. The term "non-transitory" may indicate that the storage medium is not embodied in a carrier wave or a propagated signal. However, the term "non-transitory" should not be interpreted to mean that the memory 204 is non-movable. In some examples, the memory 204 can be configured to store larger amounts of information than the memory. In certain examples, a non-transitory storage medium may store data that can, over time, change (e.g., in Random Access Memory (RAM) or cache).

The communication interface 206 can be configured to enable the electronic device 200 to connect with the at least one external entity (such as, the server, the external database, the user devices, the imaging sensors/scanners, and so on) using the communication network.

The I/O module 208 can be configured to enable the electronic device 200 to connect with at least one of the imaging sensors, scanners, cameras, and so on to capture the digital whole slide image of the stained smear samples.

The display 210 can be configured to display the enhanced visualization and localization of the HPFs that are assigned with the low density, medium density, and high density grades, the enhanced visualization and localization of the malignant and benign classes, and so on.

FIG. 2 shows exemplary blocks of the electronic device 200, but it is to be understood that other embodiments are not limited thereon. In other embodiments, the electronic device 200 may include less or more number of blocks. Further, the labels or names of the blocks are used only for illustrative purpose and does not limit the scope of the embodiments herein. One or more blocks can be combined together to perform same or substantially similar function in the electronic device 200.

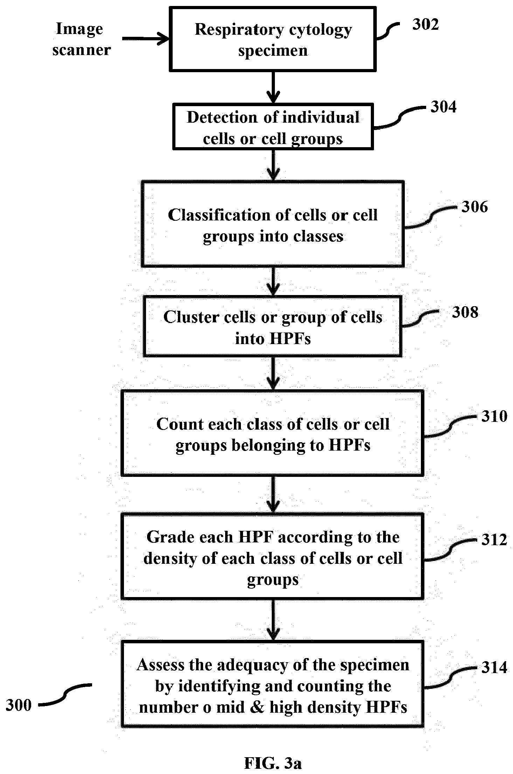

FIG. 3a is an example flow diagram depicting a method for predicting the adequacy of the respiratory cytology specimens, according to embodiments as disclosed herein. The electronic device 200 obtains (at step 302) the digital media capture of the slide, wherein the respiratory cytology specimen of the patient that is expressed over the slides for the direct smears. In an embodiment herein, the smears can be the stained smears. The electronic device 200 detects (at step 304) the individual cells/group of cells based on the obtained digital slide image of the stained smears. The electronic device 200 classifies (at step 306) the individual cells/group of cells into the classes/parameters such as lymphocyte, pigmentation, macrophages, large (atypical) cells, and so on. The electronic device 200 clusters (at step 308) the individual cells/group of cells into the HPFs based on the classification. The electronic device 200 counts (at step 310) the individual cells/group of cells belonging to the HPFs. The electronic device 200 further grades (at step 312) each HPF according to density/count of each class of the individual cell/group of cells into low, medium, high density HPFs. The electronic device 200 assesses (at step 314) the adequacy of the respiratory cytology specimens by identifying and counting the number of medium and high density HPFs.

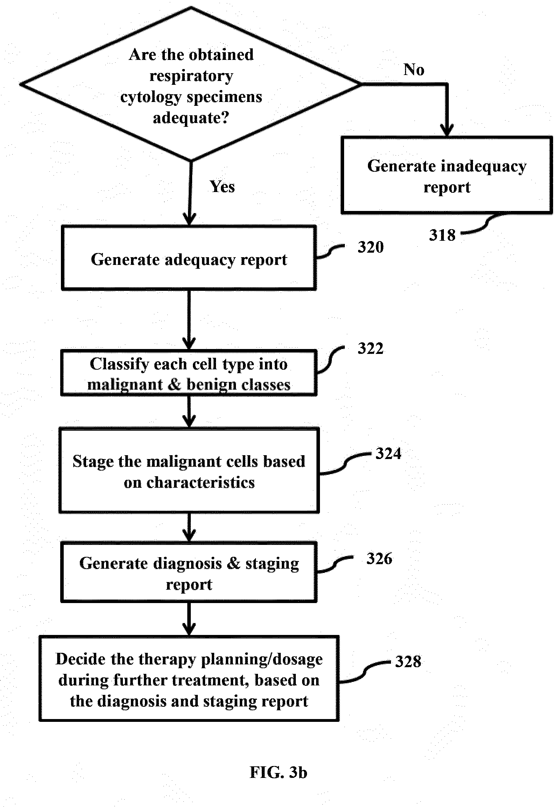

FIG. 3b is an example flow diagram depicting a method for diagnosing and staging the respiratory cytology specimens, according to embodiments as disclosed herein. Embodiments herein enable the electronic device 200 to diagnose and stage the respiratory cytology specimens based on the prediction of the adequacy of the respiratory cytology specimens. The electronic device 200 checks (at step 316) if the obtained respiratory cytology specimens are adequate for further diagnosing and staging based on assessment of the adequacy of the respiratory cytology specimens. If the obtained respiratory cytology specimens are inadequate for further diagnosing and staging, the electronic device 200 generates (at step 318) the inadequate report. If the obtained respiratory cytology specimens are adequate for further diagnosing and staging, the electronic device 200 generates (at step 320) the adequate report. The adequate report may include information about the individual cells/group of cells belonging to the HPFs that are associated with the medium density and high density and the associated types. The electronic device 200 classifies (at step 322) each cell type of the individual cells/group of cells belonging to the HPFs (such as pigmentation/macrophages, large (a typical/malignant) cells) into the malignant and benign classes.

The electronic device 200 performs staging (at step 324) of the malignant cells based on characteristics such as cell boundary, size, shape, morphology, cytoplasm to nucleus ratio, and so on. The electronic device 200 generates (at step 326) the diagnosis and the staging. The electronic device 200 further decides (at step 328) the therapy planning/dosage during further treatment, based on the diagnosis and staging report.

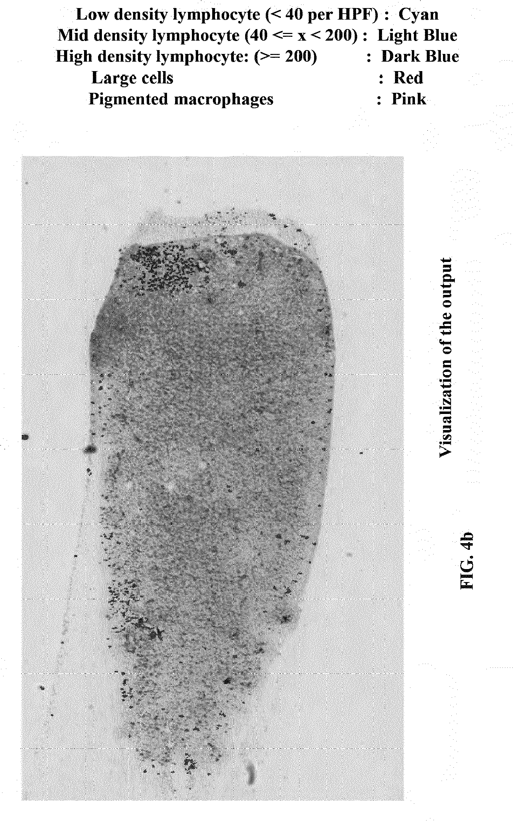

FIG. 4a is an example diagram depicting the respiratory cytology specimen of the patient received as an input for assessment, according to embodiments as disclosed herein. FIG. 4b is an example diagram depicting a visualization of an output derived by analyzing the input respiratory cytology specimen, according to embodiments as disclosed herein. The output indicates the individual cells/group of cells belonging to the HPFs of the low density (cyan), the medium density (light blue) and the high density (dark blue), the classes/clusters of the individual cells/group of cells (for example; large cells in red color, pigmented macrophages in pink color), and so on. FIG. 4c is an example diagram depicting the enhanced visualization of the output, according to embodiments as disclosed herein.

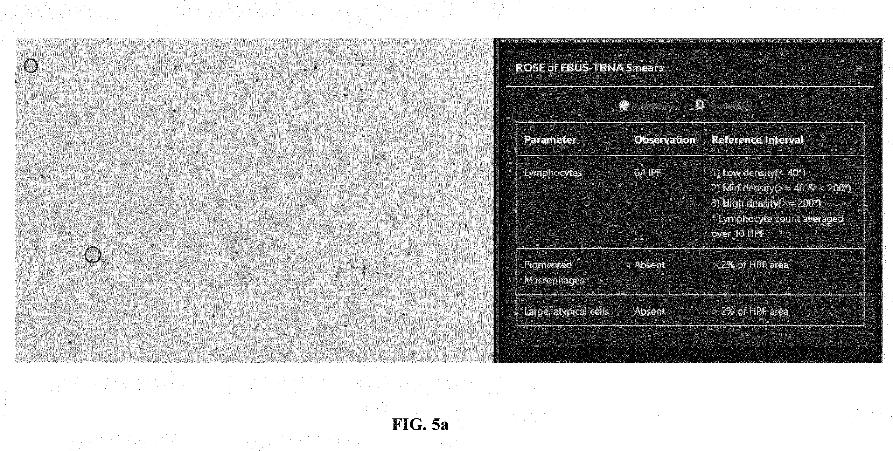

FIG. 5a is an example diagram depicting an example adequate report, according to embodiments as disclosed herein. The example adequate report includes information about the classification of the individual cells/group of cells derived from the smears (for example: rapid on-site evaluation (ROSE) of Endobronchial ultrasound-guided transbronchial needle aspiration (EBUS-TBNA) smears) into the classes/parameters (such as lymphocytes, large cells, pigmented macrophages, or the like), the classes belonging to the HPFs (observation information), reference interval, and so on.

FIG. 5b is an example diagram depicting an example inadequate report, according to embodiments as disclosed herein. The example inadequate report includes information about the classification of the individual cells/group of cells derived from the smears (for example: ROSE of EBUS-TBNA smears) into the classes/parameters (such as lymphocytes, large cells, pigmented macrophages, or the like), the classes belonging to the HPFs (observation information), reference interval, and so on.

Embodiments herein perform an automated on-site assessment of respiratory cytology specimens for reducing turnaround time for lung cancer diagnosis and staging. The assessment involves an automated assessment of adequacy of the respiratory cytology specimens and an automated diagnosis and staging of the respiratory cytology specimens.

Embodiments herein perform an accurate, reproducible, faster assessment of adequacy, diagnosis, and staging of the respiratory cytology specimens.

Embodiments herein perform the automated assessment of the respiratory cytology specimens for better patient care by avoiding repetition of invasive procedures and for increasing diagnostic yield of EBUS-TBNA procedure.

Embodiments herein perform automated quantification of adequacy grade in the respiratory cytology specimens and display a visualization of low, medium, and high lymphocyte density regions of the respiratory cytology specimens.

Embodiments herein enable an automatic generation of adequacy reports, and diagnosis and staging reports.

The embodiments disclosed herein can be implemented through at least one software program running on at least one hardware device and performing network management functions to control the elements. The elements shown in FIG. 2 can be at least one of a hardware device, or a combination of hardware device and software module.

The embodiments herein disclose methods and systems for automated assessment of respiratory cytology specimens. Therefore, it is understood that the scope of the protection is extended to such a program and in addition to a computer readable means having a message therein, such computer readable storage means contain program code means for implementation of one or more steps of the method, when the program runs on a server or mobile device or any suitable programmable device. The method is implemented in at least one embodiment through or together with a software program written in e.g. Very high speed integrated circuit Hardware Description Language (VHDL) another programming language, or implemented by one or more VHDL or several software modules being executed on at least one hardware device. The hardware device can be any kind of portable device that can be programmed. The device may also include means which could be e.g. hardware means like e.g. an ASIC, or a combination of hardware and software means, e.g. an ASIC and an FPGA, or at least one microprocessor and at least one memory with software modules located therein. The method embodiments described herein could be implemented partly in hardware and partly in software. Alternatively, the invention may be implemented on different hardware devices, e.g. using a plurality of CPUs.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of embodiments and examples, those skilled in the art will recognize that the embodiments and examples disclosed herein can be practiced with modification within the scope of the embodiments as described herein.

## Claims

1. A method for assessing a respiratory cytology specimen, the method comprising
detecting (304), by an electronic device (200), at least one of at least one individual cell and at least one group of cells in a media of a slide, wherein the slide comprises at least one smear of the respiratory cytology specimen;
classifying (306), by the electronic device (200), the at least one detected at least one individual cell and at least one group of cell into at least one class;
clustering (308), by the electronic device (200), the classified at least one detected at least one individual cell and at least one group of cell into at least one High Power Field (HPF);
counting (310), by the electronic device (200), the classified at least one detected at least one individual cell and at least one group of cell;
grading (312), by the electronic device (200), the HPF according to density/count of each class of the individual cell/group of cells into at least one of low, medium, high density HPFs; and
assessing (314), by the electronic device (200), the adequacy of the respiratory cytology specimen based on the number of medium and high density HPFs.

2. The method, as claimed in claim 1, wherein the respiratory cytology specimen of the patient is expressed over the labeled glass slides for the direct smears, wherein the smears can be the stained smears

3. The method, as claimed in claim 1, wherein the class comprises lymphocyte, pigmentation, macrophages, and large cells.

4. The method, as claimed in claim 1, wherein the method comprises
checking (316), by the electronic device (200), if the obtained respiratory cytology specimen is adequate for diagnosing and staging based on assessment of the adequacy of the respiratory cytology specimen;
generating (318), by the electronic device (200), an inadequate report, if the respiratory cytology specimen is inadequate for further diagnosing and staging; and
generating (318), by the electronic device (200), an adequate report, if the obtained respiratory cytology specimen is adequate for further diagnosing and staging, wherein the adequate report comprises information about the individual cells/group of cells belonging to the HPFs that are associated with the medium density and high density and the associated types.

5. The method, as claimed in claim 4, wherein the method comprises
classifying (322), by the electronic device (200), each cell type of at least one of the at least one individual cell and at least one group of cells belonging to the HPF into at least one of a malignant class and a benign class;
staging (324), by the electronic device (200), the cells in the malignant class, based on at least one characteristic; and
generating (326), by the electronic device (200), a diagnosis and staging report based on the staging.

6. The method, as claimed in claim 5, wherein the characteristic is at least one of cell boundary, size, shape, morphology, and cytoplasm to nucleus ratio.

7. The method, as claimed in claim 5, wherein the method comprises deciding (328), by the electronic device (200), the therapy planning/dosage during further treatment, based on the diagnosis and staging report using a regression model..

8. An electronic device (200) for assessing a respiratory cytology specimen, the electronic device configured for
detecting at least one of at least one individual cell and at least one group of cells in a media of a slide, wherein the slide comprises at least one smear of the respiratory cytology specimen;
classifying the at least one detected at least one individual cell and at least one group of cell into at least one class;
clustering the classified at least one detected at least one individual cell and at least one group of cell into at least one High Power Field (HPF);
counting the classified at least one detected at least one individual cell and at least one group of cell;
grading the HPF according to density/count of each class of the individual cell/group of cells into at least one of low, medium, high density HPFs; and
assessing the adequacy of the respiratory cytology specimen based on the number of medium and high density HPFs.

9. The electronic device, as claimed in claim 8, wherein the respiratory cytology specimen of the patient is expressed over the labeled glass slides for the direct smears, wherein the smears can be the stained smears

10. The electronic device, as claimed in claim 8, wherein the class comprises lymphocyte, pigmentation, macrophages, and large cells.

11. The electronic device, as claimed in claim 8, wherein the electronic device (200) is configured for
checking if the obtained respiratory cytology specimen is adequate for diagnosing and staging based on assessment of the adequacy of the respiratory cytology specimen;
generating an inadequate report, if the respiratory cytology specimen is inadequate for further diagnosing and staging; and
generating an adequate report, if the obtained respiratory cytology specimen is adequate for further diagnosing and staging, wherein the adequate report comprises information about the individual cells/group of cells belonging to the HPFs that are associated with the medium density and high density and the associated types.

12. The electronic device, as claimed in claim 11, wherein the electronic device (100) is configured for
classifying each cell type of at least one of the at least one individual cell and at least one group of cells belonging to the HPF into at least one of a malignant class and a benign class;
staging the cells in the malignant class, based on at least one characteristic; and generating a diagnosis and staging report based on the staging.

13. The electronic device, as claimed in claim 12, wherein the characteristic is at least one of cell boundary, size, shape, morphology, and cytoplasm to nucleus ratio.

14. The electronic device, as claimed in claim 12, wherein the electronic device (200) is further configured for determining the therapy planning/dosage during further treatment, based on the diagnosis and staging report using a regression model.
